(19) Europäisches Patentamt
European Patent Office
Office européen des brevets

(11) Publication number: **0 035 811**
**B1**

(12) **EUROPEAN PATENT SPECIFICATION**

(45) Date of publication of patent specification: **27.07.83**

(51) Int. Cl.³: **C 07 C 153/07, C 07 B 19/00**

(21) Application number: **81200234.3**

(22) Date of filing: **26.02.81**

(54) ***Process for resolving DL-S-benzoyl-beta-mercaptoisobutyric acid, and products obtained by applying this process.***

(30) Priority: **06.03.80 NL 8001341**

(43) Date of publication of application:
**16.09.81 Bulletin 81/37**

(45) Publication of the grant of the patent:
**27.07.83 Bulletin 83/30**

(84) Designated Contracting States:
**BE DE FR GB IT NL**

(56) References cited:
**US - A - 4 224 457**

**LIEBIGS ANNALEN DER CHEMIE, vol. 757 (1972), H. KAEHLER et al. "Die Spaltung racemischer Carbonylverbindungen über bifunktionelle Reagenzien mittels optisch aktiver Basen" pages 15—22.**

**CHEMICAL PHARMACEUTICAL BULLETIN, vol. 26, no. 4, April 1978 ITARU MITA et al. "New sulfhydryl compounds with potent antihypertensive activities" pages 1333—1335.**

(73) Proprietor: **Océ-Andeno B.V.
Grubbenvorsterweg 8
NL-5928 NX Venlo (NL)**

(72) Inventor: **de Heij, Nicolaas, Antonius
Wilgenstraat 22
Venlo (NL)**

(74) Representative: **Bleukx, Lucas Lodewijk Maria, Ir. et al,
Océ-Nederland B.V. Patents & Information Dept.
Postbus 101
NL-5900 MA Venlo (NL)**

**EP 0 035 811 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Process for resolving DL-S-benzoyl-β-mercaptoisobutyric acid, and products obtained by applying this process

The invention relates to a process for resolving DL-S-benzoyl-β-mercaptoisobutyric acid, to D(—)-S-benzoyl-β-mercaptoisobutyric acid itself as well as to its salts, esters or derivatives obtained by applying the process according to the invention.

DL-S-benzoyl-β-mercaptoisobytyric acid, its preparation, for example, by reacting thiobenzoic acid with methacrylic acid and the resolution of DS-S-benzoyl-β-mercaptoisobutyric acid into its optical antipodes are already disclosed in European Patent Application 8833 which has been published on March 19, 1980. Said European Patent Application describes a process for the preparation of compounds of formula

$$R{-}\underset{\underset{O}{\|}}{C}{-}S{-}CH_2{-}CH(CH_3){-}COOH,$$

in which R can have one of the meanings indicated in that Application. According to that process the compounds can be prepared by resolving a compound of the formula referred to above, in which R represents a phenyl group, by means of cinchonidine, D-(—)-2-aminobutanol-1 or a derivative of the latter and then, if so desired, re-acylating it.

D(—)-S-benzoyl-β-mercaptoisobutyric acid is a very suitable starting product for the preparation of 1-(3-mercapto-2-D-methyl propanoyl)-L-proline and its derivatives, which are biologically active compounds having interesting pharmaceutical properties, such as their lowering effect on certain forms of high blood pressure.

Though the resolving process described in our afore-mentioned Application is quite useful and gives reasonable yields, in view of the promising pharmaceutical properties of the above-mentioned proline compounds, the want of a process that simplifies the resolution of DL-S-benzoyl-β-mercaptoisobutyric acid and permits a higher yield, so a more economical way, remained.

The present invention meets this want. Therefore, it relates to a process of the kind referred to in the opening paragraph of this Application, which process is characterized in that the resolution is carried out by means of D-(+)-N-benzyl-α-phenethylamine.

D-(+)-N-benzyl-α-phenethylamine (hereinafter referred to as NBPA) is a compound known per se, which according to the literature can be prepared from optically active D-α-phenethylamine and benzyl chloride. Although suggestions as to the usefulness of NBPA as a resolving agent existed already, this compound does not belong to the series of well-known resolving agents. In S. H. Wilen's book: "Resolving agents and resolutions in organic chemistry" (1971), which is specially devoted to this subject, this compound does not even appear at all.

It has now been found that the resolution of DL-S-benzoyl-β-mercaptoisobutyric acid using NBPA can be realized more simply and with a considerably higher yield than with the resolving process used so far. This is the case, in particular, if:

a) a solution of DL-S-benzoyl-β-mercaptoisobutyric acid in isopropanol is prepared,

b) to this solution, NBPA is added as resolving agent,

c) the salt formed from the resolving agent with D-(—)-S-benzoyl-β-mercaptoisobutyric acid is crystallised out and isolated,

d) if necessary, the optical purity of this salt is improved by recrystallization,

e) the pure salt is subjected to a basic hydrolysis process, in order to remove the resolving agent, followed by acidifying the separated aqueous solution,

f) the liberated D-(—)-S-benzoyl-β-mercaptoisobutyric acid is won and, if necessary, purified.

Although other solvents than isopropanol, such as ethanol, n-butanol, toluene, acetone, ethyl acetate, t-butyl acetate and di-isopropyl ether are useful, the use of isopropanol is preferred.

The invention is also directed, of course, to the products obtained by applying the present process. In this connection, the D-(—)-S-benzoyl-β-mercaptoisobutyric acid itself as well as its salts, esters or derivatives may be thought of.

As salts, in addition to the NBPA-salt, of course, the ammonium salts and alkaline (earth) metal salts such as the Na-, K-, Ca-, Ba-, and Mg-salt may be considered. In case of esters, simple esters such as the lower alkyl esters and, in case of derivatives, related simple compounds may be thought of.

The following examples may serve to illustrate the invention:

## Example 1

*a.* Salification

A solution of 1 mol of DL-S-benzoyl-β-mercaptoisobutyric acid was prepared in 500 ml of isopropanol. To this solution 0.95 mol of NBPA was added. The resulting mixture was heated to about 45°C. After stirring the solution for half an hour at this temperature it was cooled, with stirring, to 20°C. After additional stirring for about 3 hours at that temperature, the crystallized salt was centrifuged, washed with approximately 50 ml of isopropanol and dried.

The yield was 217 g, which corresponds to approximately 50%; melting point: 104—106°C.

*b.* Recrystallisation of the salt

The 217 g of salt obtained above were added to 500 ml of isopropanol, after which the mixture was heated, with stirring, to approximately 70°C. At that temperature the salt dissolved completely. Stirring all the time, the solution was cooled slowly. Some seed crystals were added at 65°C, after which the solution was further cooled to 20°C in about an hour's time and the stirring was prolonged at this temperature for approximately 2 hours.

The crystallized salt was certrifuged, washed with 50 ml of isopropanol and dried. The yield was 180 g (=approximately 41.5%); melting point: 107—108°C and $[\alpha]_D^{22} = -3°$ to $-4.4°$ (1% in 96% ethanol).

*c.* Liberation of the D(—)-isomer from the NBPA-salt

The 180 g of purified salt obtained by the process *b* were added to a stirred mixture of 200 ml of 1,2-dichloroethane, 53 g of soda and 500 ml of water. After vigorous stirring for one hour the dichloroethane layer was separated from the aqueous layer. The first layer contained the NBPA, the second the desired acid in the form of the sodium salt. With stirring, concentrated hydrochloric acid was added to the aqueous layer till a pH-value of 1 was reached. The precipitated D-(—)-S-benzoyl-β-mercapto-isobutyric acid was filtered over a Büchner funnel, washed with water and dried. The overall yield was 40%; melting point: 67—69°C and $[\alpha]_D^{22} = -43°$ to $-44°$ (1% in 96% ethanol).

The greater part of the NBPA started from could be recovered from the various mother liquors. Moreover the various isopropanol layers obtained during the working up process, which layers still contained remainders of the NBPA-salt of D-(—)-S-benzoyl-β-mercaptoisobutyric acid, could be reused.

Compared with the overall yield recorded in our previous European Patent Application 79200477.2 (22.4% in Example 1 and 30.7% in Example 2), the resolution according to the present invention gives considerably higher yields.

Additional advantages are that, generally, the reaction time is shorter and the number of purification steps smaller. Further, a strongly marked advantage lies in that it is possible to work in considerably more concentrated solutions. Thus, according to the Examples of our former patent application the solvent required per one mol of DL-compound or resolving agent amounts to 5,100 and 3,000 ml, respectively, whereas according to the present invention an amount of 500 ml suffices.

Another advantage is that the resolution of the DL-S-benzoyl-β-mercaptoisobutyric acid can be carried out directly after its preparation from thiobenzoic acid and methacrylic acid, *id est*, without the necessity of isolating first the DL-compound, which was not practicable, or scarcely so, when the resolving agents referred to above were used.

The following example may serve to illustrate this.

Example 2

With stirring, 1.05 mol of thiobenzoic acid were added to 250 ml of isopropanol. The solution was heated to about 80°C and 1.00 mol of methacrylic acid was added in about ½ hour's time. The solution was heated to reflux temperature. After refluxing the solution for 2 hours it was cooled again to 40°C, and 250 ml of isopropanol, 0.95 mol of NBPA and some seed crystals of the desired salt were added.

The mixture was stirred for ½ hour at 40°C, was then cooled to 20°C, whereupon the stirring was continued for 4 hours at this temperature. The salt, which had meanwhile crystallized out, was centrifuged, washed with approximately 50 ml of isopropanol and dried. The yield was approximately 50%.

The salt obtained was further treated as described in Example 1 under *b* and *c*. The overall yield of D-(—)-S-benzoyl-β-mercapto-isobutyric acid was 39%; melting point: 67—69°C.

Finally, the use of NBPA has the additional advantage that it can be recovered in a considerably simpler way than the resolving agents used before.

The recovery of the NBPA occurred as follows:

The mother liquor of the resolving step was concentrated by evaporation, in which process the isopropanol was recovered. The residue was treated with 1,2-dichloroethane and diluted sodium hydroxide. After vigorous stirring for half an hour the dichloroethane layer was separated, washed with diluted sodium hydroxide and twice with water. The dichloroethane layer was dried, the dichloroethane was evaporated and the NBPA was recovered in a practically quantitative yield. The mother liquor of the recrystallisation step could be reused for a next resolution or recrystallisation. From the dichloroethane layer liberated during the hydrolysis step the NBPA could be recovered quantitatively by washing the layer with diluted lye and water and evaporating the dichloroethane.

**Claims**

1. Process for resolving DL-S-benzoyl-β-mercaptoisobutyric acid by means of a resolving agent, characterized in that the resolution is carried out by means of D-(+)-N-benzyl-α-phenethylamine.

2. Process according to claim 1, characterized in that

a) a solution of DL-S-benzoyl-β-mercapto-isobutyric acid in isopropanol is prepared,

b) to this solution, D-(+)-N-benzyl-α-phenethylamine is added as resolving agent,

c) the salt formed from the resolving agent

with D-(—)-S-benzoyl-$\beta$-mercaptoisobutyric acid is crystallised out and isolated,

d) if necessary, the optical purity of this salt is improved by recrystallization,

e) the pure salt is subjected to a basic hydrolysis process, in order to remove the resolving agent, followed by acidifying the separated aqueous solution, and

f) the liberated D-(—)-S-benzoyl-$\beta$-mercapto-isobutyric acid is won and, if necessary, purified.

**Revendications**

1. Procédé de résolution de l'acide DL-S-benzoyl-$\beta$-mercaptoisobutyrique au moyen d'un agent de résolution caractérisé en ce qu'on effectue la résolution au moyen de la D-(+)-N-benzyl-$\alpha$-phénéthylamine.

2. Procédé selon la revendication 1, caractérisé en ce que

a) on prépare une solution d'acide DL-S-benzoyl-$\beta$-mercapto-isobutyrique dans l'isopropanol,

b) on ajoute à cette solution de la D-(+)-N-benzyl-$\alpha$-phénéthylamine comme agent de résolution,

c) on cristallise et isole le sel formé à partir de l'agent de résolution avec l'acide D-(—)-S-benzoyl-$\beta$-mercapto-isobutyrique,

d) si nécessaire on eméliore la pureté optique ce ce sel par recristallisation,

e) on soumet le sel pur à une opération d'hydrolyse basique, pour éliminer l'agent de résolution, puis on acidifie la solution aqueuse séparée, et

f) on recueille l'acide D-(—)-S-benzoyl-$\beta$-mercapto-isobutyrique libéré et, si nécessaire, on le purifie.

**Patentansprüche**

1. Verfahren zur optischen Spaltung von DL-S-Benzoyl-$\beta$-merkaptoisobuttersäure mit Hilfe eines Spaltmittels, dadurch gekennzeichnet, dass man die Spaltung mit Hilfe von D-(+)-N-Benzoyl-$\alpha$-phenäthylamin durchführt.

2. Verfahren nach Anspruch 1, dadurch gekennzeichnet, dass man

a) eine Lösung von DL-S-Benzoyl-$\beta$-merkapto-isobuttersäure in Isopropanol herstellt,

b) zu dieser Lösung D-(+)-N-Benzyl-$\alpha$-phenäthylamin als Spaltmittel gibt,

c) das vom Spaltmittel gebildete Salz mit D-(—)-S-Benzoyl-$\beta$-merkaptoisobuttersäure auskristallisieren lässt und isoliert,

d) die optische Reinheit dieses Salzes nötigenfalls durch Umkristallisation erhöht,

e) zur Entfernung des Spaltmittels das reine Salz einer basischer Hydrolyse unterzieht und darauf die wässerige Lösung nach deren Abtrennung ansäuert, und

f) die frei gemachte D-(—)-S-Benzoyl-$\beta$-merkapto-isobuttersäure gewinnt und nötigenfalls reinigt.